(19) Europäisches Patentamt
European Patent Office
Office européen des brevets

(11) Veröffentlichungsnummer: **0 308 820 B1**

(12) **EUROPÄISCHE PATENTSCHRIFT**

(45) Veröffentlichungstag der Patentschrift: **27.11.91**

(51) Int. Cl.[5]: **C07C 311/15**, C23F 11/16, C07D 211/62

(21) Anmeldenummer: **88115163.3**

(22) Anmeldetag: **16.09.88**

# (54) Zink-, Blei- und/oder Calciumsalze von Carbonsäuren und deren Verwendung als Korrosionsschutzmittel.

(30) Priorität: **25.09.87 DE 3732374**

(43) Veröffentlichungstag der Anmeldung:
**29.03.89 Patentblatt 89/13**

(45) Bekanntmachung des Hinweises auf die Patenterteilung:
**27.11.91 Patentblatt 91/48**

(84) Benannte Vertragsstaaten:
**AT BE CH DE ES FR GB IT LI NL**

(56) Entgegenhaltungen:
**EP-A- 0 068 407**
**DE-A- 2 947 418**
**DE-A- 3 616 721**

(73) Patentinhaber: **BASF Lacke + Farben Aktiengesellschaft**
**Max-Winkelmann-Strasse 80**
**W-4400 Münster(DE)**

(72) Erfinder: **Liedek, Egon, Dr.**
**Holunder-Weg 16**
**7300 Esslingen(DE)**
Erfinder: **Haegele, Gerhard, Dr.**
**Arnold-Cahn-Weg 10**
**W-7000 Stuttgart 50(DE)**

(74) Vertreter: **Wurzler, Hartmut, Dr.**
**i.Fa. BASF Aktiengesellschaft Carl-Bosch-Strasse 38**
**W-6700 Ludwigshafen(DE)**

EP 0 308 820 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Es wurden schon zahlreiche Produkte vorgeschlagen, die Mennige und Pigmente auf der Basis Zinkchromat als Korrosionsinhibitoren ersetzen sollen. Diese Ersatzprodukte enthalten in der Regel metallhaltige organische Verbindungen.

So werden z.B. in der DE-C-25 02 781 Zink- und Bleisalze und Gemische dieser Salze auf der Basis von 5-Nitroisophthalsäure als rostschützende Zusätze für Anstrichmittel beschrieben. Von den in dieser Patentschrift beschriebenen Salzen wird vor allem das Zinksalz der 5-Nitroisophthalsäure technisch angewendet. Ein Nachteil der in der DE-C-25 02 781 beschriebenen Produkte ist jedoch, daß ihre Wirkung und Anwendungsbreite begrenzt sind. So wird z.B. bemängelt, daß eine ausreichende Wirkung in wäßrigen Anstrichsystemen nicht vorhanden ist.

Ähnlich verhalten sich die Zink- und Bleisalze, die in den DE-A-28 07 698 und 28 24 508 beschrieben werden.

Zur Bearbeitung der sich laufend ändernden und vielschichtigen Problematik der Rostinhibierung mit Hilfe von Anstrichmitteln ist die Bereitstellung weiterer aktiver Substanzen mit in verschiedener Richtung verbesserten Eigenschaften erwünscht. Bereits das Angebot weiterer aktiver Substanzen, die im Vergleich zum Stand der Technik als gleichwertige Alternativen anzusehen sind, kann technisch fortschrittlich sein, da oft erst umfangreiche Erfahrungen bei spezifischen technischen Anwendungen Vorteile erkennen lassen.

Aufgabe der vorliegenden Erfindung war es, weitere für den Korrosionsschutz geeignete Pigmente bereitzustellen, die nicht die Nachteile der bekannten Korrosionsschutzpigmente aufweisen.

Die Erfindung betrifft Zink-, Blei-, Calcium-Salze oder gemischte Salze mit diesen Metallen von Carbonsäuren der allgemeinen Formel

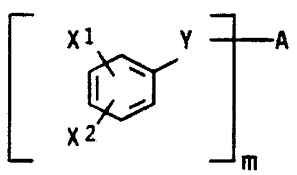

(I),

in der A für einen Rest der Formel

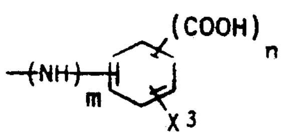

(II)

oder

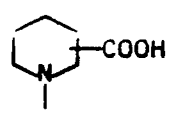

(III)

- Y für -CO- oder $-SO_2-$,
- $X^1$ für $C_1$- bis $C_8$-Alkyl, $C_1$- bis $C_8$-Alkoxy oder Halogen,
- $X^2$ für Wasserstoff, $C_1$- bis $C_8$-Alkyl oder Halogen,
- $X^3$ für Wasserstoff, Nitro oder Halogen,
- m für 1 oder 2 und
- n für 1 oder 2 stehen, und wobei $X^1$ auch Wasserstoff sein kann, wenn A entweder ein Rest der Formel (III) oder $X^3$ Nitro oder m = 2 ist.

Die Salze der Erfindung weisen im Vergleich zu den nächstliegenden aus der DE-C-25 02 781 bekannten Zink- bzw. Bleisalzen in nicht wäßrigen Anstrichsystemen, z.B. in Anstrichmitteln aus lufttrocknenden Leinöl/Holzöl-Alkydharzen bessere Korrosionsschutzwerte auf. Außerdem sind die Zink- und Bleisalze gemäß der vorliegenden Erfindung auch für wäßrige Anstrichsysteme geeignet, z.B. für wasserverdünnbare Alkydharze.

Als $C_1$- bis $C_8$-Alkyl kommen für $X^1$ und $X^2$ im einzelnen z.B. in Betracht: Methyl, Ethyl, n- und i-Propyl, n-, i- und tert-Butyl, n- und i-Amyl, 1,1-Dimethylpropyl-1 und tert-Amyl, n- und i-Hexyl, Heptyl, n- und i-Octyl und 2-Ethylhexyl.

Für $X^1$ sind als Alkyl $C_1$- bis $C_4$-Alkyl bevorzugt, insbesondere Methyl und Ethyl.

Als $C_1$- bis $C_8$-Alkoxy sind z.B. Methoxy, Ethoxy, Propoxy, Butoxy, Pentoxy und Octoxy zu nennen, von denen die $C_1$- bis $C_4$-Alkoxyreste bevorzugt sind.

Als Halogen sind für $X^1$, $X^2$ und/oder $X^3$ Fluor, Brom und Chlor zu nennen; von diesen ist Chlor bevorzugt.

Bevorzugt sind aufgrund ihrer Eigenschaften Zink-, Blei- und/oder Calciumsalze von Carbonsäuren (I), in denen Y $-SO_2-$ bedeutet.

Von diesen sind die Carbonsäuren (I) hervorzuheben, in denen $X^1$ für $C_1$- bis $C_4$-Alkyl, $C_1$- bis $C_4$-Alkoxy oder Chlor, $X^2$ und $X^3$ für Wasserstoff und n und m jeweils für 1 stehen.

Besonders bevorzugt sind Verbindungen (I), in denen $X^2$ und $X^3$ Wasserstoff, n = m = 1 und $X^1$ Methyl oder Ethyl sind.

Ganz besonders bevorzugt sind Zink-, Blei- und/oder Calciumsalze von Carbonsäuren der Formel

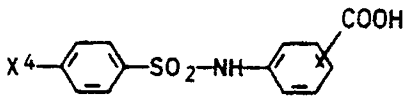

(IV),

in der $X^4$ Ethyl, vorzugsweise Methyl ist und die Carboxylgruppe in 2-, 3- oder 4-Stellung steht.

Weiterhin sind Blei-, Zink- und/oder Calciumsalze der Carbonsäure

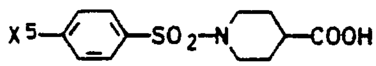

(V),

zu nennen, in der $X^5$ Wasserstoff oder $C_1$- bis $C_4$-Alkyl, vorzugsweise Methyl, Ethyl und insbesondere Wasserstoff ist.

Ganz besonders bevorzugt sind basische Zink- und/oder Bleisalze der Carbonsäuren (I), (III) und (IV), bei denen das Verhältnis Carbonsäure zu Zink und/oder Blei etwa 1 zu 1 Mol beträgt.

Diese basischen Salze entsprechen bei den Carbonsäuren (IV) und (V) den Formeln

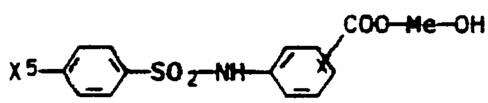

(IVa),

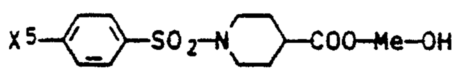

(Va),

worin Me für Zn und/oder Pb steht.

Die Calciumsalze von (I), bzw. (IV) und (V) enthalten vorzugsweise 2 Mol (I), (IV) oder (V) je Mol Calcium.

Die neuen Salze sind in den flüssigen Anstrichmitteln im allgemeinen in Mengen zwischen 0,2 und 5, vorzugsweise zwischen 0,5 und 3 Gew.%, bezogen auf den Feststoff, enthalten.

Die neuen Zink-, Blei-, Calcium-Salze oder die gemischten Salze mit diesen Metallen werden in an sich bekannter Weise entweder durch Umsetzen der Alkalimetallsalze der Säure (I) mit löslichen Calcium-, Zink-, Bleisalzen oder deren Gemischen, wie Zinksulfat, Calcium- und/oder Bleinitrat oder durch Umsetzen der freien Säure (I) mit Zinkoxid, Bleioxid und/oder Calciumoxid in wäßrigem Medium in der Wärme erhalten.

Durch Variation des Molverhältnisses Carbonsäure (I) zu Zink-, Blei-, Calciumverbindungen oder Gemischen davon, der Konzentration, der Temperatur, der Zeit und des pH-Wertes werden einheitliche d.h. definierte Zink-, Blei- bzw. Zink/Bleiverbindungen mit unterschiedlicher Basizität erhalten.

Im Falle der Umsetzung von (I) mit Calciumverbindungen erhält man die neutralen Salze.

Die Neutralsalze von (I) erhält man in der Regel bei einem Verhältnis von 2 Äquivalenten Carbonsäure und 2 Äquivalenten (= 1 Mol) Zink-, Blei- und/oder Calciumverbindung in neutralem Reaktionsmedium. Zur Herstellung der basischen Salze wendet man je Äquivalent Carbonsäure zwei Äquivalente (= 1 Mol) Zink- und/oder Bleioxid an, wobei der dabei resultierende höhere pH-Wert zur Bildung der basischen Salze ausreicht. Verwendet man anstelle der Oxide die entsprechenden wasserlöslichen Salze, muß dem Gemisch die äquivalente Menge an Basen, d.h. 2 Äquivalente je Äquivalent Carbonsäure zugegeben werden, so daß die Bildung der basischen Salze im alkalischen Bereich erfolgt. Bei Verwendung von löslichen Salzen der

Metalle wird die alkalische Lösung der Säure zweckmäßigerweise vorgelegt.

Als Basen kommen vor allem die Alkalimetallhydroxide wie Natrium- und Kaliumhydroxid in Form der wäßrigen Lösungen in Betracht.

Das wäßrige Reaktionsgemisch wird solange bei der gewünschten Temperatur gehalten bis der pH-Wert der wäßrigen Phase konstant bleibt. Die ausgefallenen Salze von (I) werden in üblicher Weise isoliert, gewaschen und getrocknet.

Die Beurteilung, welche Verbindung entstanden ist, erfolgt im allgemeinen durch Vergleich der Analysendaten, der IR-Spektren und der Röntgendiffraktogramme. In der Regel läßt sich nicht Vorhersagen, ob von einer organischen Säure stabile basische Zink- und/oder Bleisalze hergestellt werden können.

Die Herstellung der Salze ist in den Beispielen beschrieben.

I. Ausführungsbeispiele

Beispiel 1

14,57 g (0,05M) N-Toluolsulfonylanthranilsäure wurden in 175 ml Wasser unter Zusatz von 25 ml 4n-NaOH unter leichtem Erwärmen gelöst. Dann wurde eine Lösung aus 14,38 g (0,05M) $ZnSO_4.7H_2O$ in 50 ml Wasser langsam zugegeben, die entstandene Suspension auf 400 ml verdünnt und 4 Stunden bei 30 °C weitergerührt bis der pH-Wert konstant blieb: pH = 8,5. Das Produkt wurde abgesaugt, gewaschen und bei 80 °C getrocknet. Ausbeute 17,8 g eines Produkts mit 1,14 Mol Zn pro Mol Ausgangssäure. Im Röntgendiffraktogramm war freies ZnO nicht sichtbar.

Beispiel 2

14,57 g (0,05M) N-Toluolsulfonylamino-3-benzoesäure wurden in 250 ml Wasser unter Zusatz von 25 ml 4n-NaOH bei 25 °C gelöst. Dann wurden unter Rühren 14,38 g (0,05M) $ZnSO_4.7H_2O$ gelöst in 50 ml Wasser langsam zugegeben, die entstandene Suspension auf 450 ml verdünnt und bei 75 °C ca. 3 Stunden weitergerührt. Nach dem Kaltrühren (pH = 7,5), Absaugen, Waschen und Trocknen bei 80 °C erhielt man 15,2 g einer Substanz, die pro Mol Ausgangssäure ca. 1,3 Mol Zn enthielt.

Beispiel 3

14,57 g (0,05M) N-Toluolsulfonylamino-4-benzoesäure wurden in 250 ml Wasser unter Zusatz von 25 ml 4n-NaOH bei 25 °C gelöst. Dann wurden 14,38 g (0,05M) $ZnSO_4.7H_2O$ gelöst in 50 ml Wasser langsam unter Rühren zugegeben. Nach dem Verdünnen auf 450 ml Gesamtvolumen wurde langsam auf 80 °C erwärmt und diese Temperatur unter Rühren 4 Std. gehalten, wobei sich ein pH-Wert von 6,3 einstellte. Nach dem Kaltrühren (23 °C, pH = 7,3) und Aufarbeiten wie bei Beispiel 3 resultierten 14,9 g eines Produkts, das 1,36 Mol Zn pro Mol Ausgangssäure enthielt.

Beispiel 4

Bas. Zn-N-Benzolsulfonyl-piperidin-4-carboxylat.

12,12 g (0,045M) N-Benzolsulfonyl-piperidin-4-carbonsäure wurden in 250 ml Wasser unter Zusatz von 22,5 ml 4n-NaOH bei 50 °C gelöst. Dann wurden 12,94 g (0,045M) $ZnSO_4.7H_2O$ gelöst in 100 ml Wasser bei 50 °C langsam zugegeben. Nach 3 1/2 stündigem Rühren bei 50 °C stellte sich der pH-Wert auf 7,3 ein. Nun wurde kaltgerührt, abgesaugt, gewaschen und getrocknet. Es resultierten 15,8 g eines Produkts, das 1,05 Mol Zn pro Mol Ausgangssäure enthielt.

Beispiel 5

Bas. Zn-3,5-Di(benzolsulfonylamino)-benzoat.

10,81 g (0,025M) 3,5-Di(benzolsulfonylamino)-benzoesäure wurden in 300 ml Wasser unter Zusatz von 12,5 ml 4n-NaOH gelöst. Anschließend wurden 7,19 g (0,025M) $ZnSO_4.7H_2O$ gelöst in 50 ml Wasser langsam Zugegeben. Die entstandene Suspension wurde 3 Stunden bei 23 °C und weitere 3 Stunden bei 75 °C gerührt. Der Niederschlag wurde abgesaugt, gewaschen und bei 80 °C getrocknet. Ausbeute 11,4 g.

Beispiel 6

10,06 g (0,03 M) 5-Toluolsulfonylamino-isophthalsäure wurden in 150 ml Wasser unter Zusatz von 30 ml 4n-NaOH gelöst. Nach Erwärmen auf 80 °C wurden 17,25 g (0,06 M) $ZnSO_4.7H_2O$ in 80 ml Wasser langsam zugegeben. Die entstandene Suspension wurde unter Rühren 3 Stunden bei 80 °C gehalten. Nach Kaltrühren, Absaugen, Waschen und Trocknen resultierten 12,4 g einer farblosen, ZnO-freien Substanz, die bei der Prüfung nach II einen Korrosionsschutzwert von 72 ergab.

Beispiel 7

Ca-N-(p-Tcluolsulfonyl)-anthranilat

14,56 g (0,05 M) N-p-Toluolsulfonyl-anthranilsäure in 50 ml Wasser wurden unter Zusatz von 50 ml n-NaOH gelöst. Anschließend wurden 5,9 g (0,025 M) $Ca(NO_3)_2.4H_2O$ in 50 ml Wasser langsam unter Rühren zugegeben. Nach Absaugen, Waschen und Trocknen des entstandenen Niederschlags resultierten 13,3 g des gewünschten Ca-Salzes.

II. Anwendungstechnische Prüfung

Die Ermittlung des Korrosionsschutzwertes der Korrosionsschutzpigmente erfolgte in nachstehender Weise:
Zunächst wird ein Grundanstrichmittel mit einer Pigmentvolumenkonzentration (PVK) von ca 36 % durch zweistündiges Dispergieren mit 2mm-Glasperlen auf einem Schütteldispergiergerät nach folgender Rezeptur hergestellt:

| | |
|---|---|
| 1,5 Gew.-Teile | Testsubstanz, |
| 16,5 Gew.-Teile | Mikrotalkum, |
| 12,0 Gew.-Teile | mikronisiertes Calciumkarbonat, |
| 10,0 Gew.-Teile | Eisenoxidrotpigment und |
| 75,0 Gew.-Teile | Bindemittellösung, die 32 Gew.% eines harzmodifizierten Leinöl-/Holzöl-Alkydharzes mit einer Säurezahl <25 enthält (Alftalat® AM 380 der Farbw.Hoechst AG), |

Mit Hilfe einer Lackschleuder werden ungebonderte, entfettete Stahl-Tiefziehbleche (USt 1405) in der Weise beschichtet, daß nach 7 Tagen Lufttrocknung und 2 Stunden Nachtrocknung bei 50 °C mittlere Schichtdicken von 40 µm resultieren. Die Testanstriche werden definiert angeritzt und nach 400 Stunden Salzsprühbelastung (DIN 50021) verglichen.

Die Bewertung erfolgt nach einem kombinierten System, bei dem der Korrosionszustand des abgelaugten Anstrichuntergrunds, die Unterrostung am Ritz, sowie die Enthaftung (ermittelt durch einen Klebestreifen-Abziehtest) einbezogen sind.

Als Ergebnis resultiert ein Korrosionsschutzwert (KW), der zwischen KW Null (= dem Anstrichmittel ohne Testsubstanz) und dem theoretisch unveränderten Anstrich mit KW 100 liegt. Für die Praxis sind Korrosionsschutzwerte um 50 als gut und Werte über 70 als sehr gut zu bezeichnen.

In der oben angegebenen Weise wurden mit den Salzen der Beispiele 1 bis 5 Anstrichmittel hergestellt. Die beschichteten Bleche wurden nach DIN 50021 getestet und nach 400 Stunden beurteilt.

Die angewandten Mengen an Salz und der KW-Wert sind in der folgenden Tabelle zusammengestellt.

Tabelle

Zusammenstellung der Ergebnisse der Korrosionsschutzprüfung.

| Salz des Beispiels | Menge [Gew.-Teile] | Korrosionsschutzwert nach 400 h erfindungsgemäß | Stand der Technik 1) |
|---|---|---|---|
| 1 | 1,5 | 65 | 51 |
| 2 | 1,5 | 73 | 47 |
| 3 | 1,5 | 76 | 47 |
| 4 | 1,5 | 77 | 71 |
| 5 | 1,5 | 74 | 63 |

1) Zink-5-Nitroisophthalat nach DE-C-25 02 781

Das nach Beispiel 7 hergestellte Ca-Salz wurde wie oben unter II angegeben geprüft, jedoch wurden anstelle von 1,5 Gew.-Teilen ein Gemisch aus 0,85 Gew.-Teilen basischem Zink-5-nitroisophthalat der DE-C-25 02 781 und 0,15 Gew.-Teilen des Ca-Salzes aus Beispiel 7 verwendet.

Zum Vergleich wurde ein Schutzmittel, das nur Zink-5-nitroisophthalat des Standes der Technik enthält, unter gleichen Bedingungen mitgeprüft.

| Anstrich enthält | Korrosionsschutzwert nach 400 h |
|---|---|
| a) 1,5 Gew.-Teile bas. Zink-5-nitroisophthalat | 54 |
| b) 1,0 Gew.-Teile " | 44 |
| c) 0,85 Gew.-Teile " + 0,15 Gew.-Teile Ca-Salz Beispiel 7 | 57 |

Die Ergebnisse zeigen, daß bei Mitverwendung der Ca-Verbindung des Beispiels 7 die Menge an Korrosionsschutzmittel ohne Verlust in der Wirkung verringert werden kann.

**Patentansprüche**

**Patentansprüche für folgende Vertragsstaaten : AT, BE, CH, DE, FR, GB, IT, LI, NL**

1. Zink-, Blei-, Calcium-Salze oder zwei oder drei dieser Metalle enthaltende gemischte Salze von Carbonsäuren der allgemeinen Formel

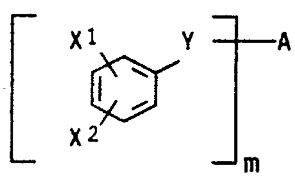

(I),

in der A für einen Rest der Formel

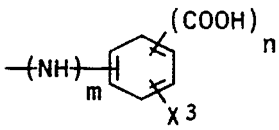 oder 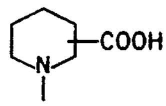 ,

(II) (III)

| | |
|---|---|
| Y | für -CO- oder $-SO_2-$, |
| $X^1$ | für $C_1$- bis $C_8$-Alkyl, $C_1$- bis $C_8$-Alkoxy oder Halogen, |
| $X^2$ | für Wasserstoff, $C_1$- bis $C_8$-Alkyl oder Halogen, |
| $X^3$ | für Wasserstoff, Nitro oder Halogen, |
| m | für 1 oder 2 und |
| n | für 1 oder 2 stehen, und wobei $X^1$ auch Wasserstoff sein kann, wenn A entweder ein Rest der Formel (III) oder $X^3$ Nitro oder m = 2 ist. |

**2.** Zink-, Blei-, Calcium-Salze oder gemischte Salze davon gemäß Anspruch 1, dadurch gekennzeichnet, daß Y = $-SO_2-$ ist.

**3.** Zink-, Blei-, Calcium-Salze oder gemischte Salze davon gemäß Anspruch 1 oder 2, dadurch gekennzeichnet, daß $X^1$ für $C_1$- bis $C_4$-Alkyl, $C_1$- bis $C_4$-Alkoxy oder Chlor, $X^2$ für Wasserstoff und n und m jeweils für 1 stehen.

**4.** Zink-, Blei-, Calcium-Salze oder gemischte Salze davon gemäß Anspruch 3, dadurch gekennzeichnet, daß $X^1$ für Methyl oder Ethyl steht.

**5.** Zink-, Blei- oder Zink-Blei-Salze gemäß Anspruch 1, 2, 3 oder 4, dadurch gekennzeichnet, daß das Verhältnis Carbonsäure zu Zink und/oder Blei etwa 1:1 Mol beträgt.

**6.** Calciumsalze gemäß Anspruch 1, 2, 3 oder 4, dadurch gekennzeichnet, daß das Verhältnis Carbonsäure zu Calcium etwa 2:1 Mol beträgt.

**7.** Zink-, Blei-, Calcium-Salze oder gemischte Salze davon gemäß Anspruch 1, dadurch gekennzeichnet, daß die Carbonsäure (I) den Formeln

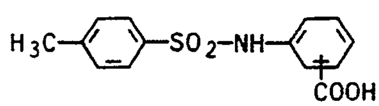 ,

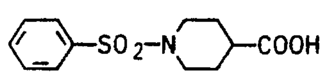 oder

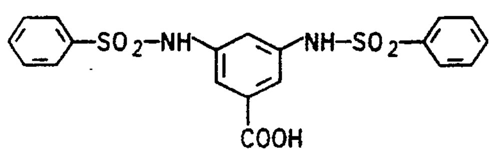

entspricht.

**8.** Zink-, Blei- oder Zink/Bleisalze gemäß Anspruch 7, dadurch gekennzeichnet, daß das Verhältnis Carbonsäure zu Zink und/oder Blei etwa 1:1 Mol beträgt.

**9.** Calciumsalze gemäß Anspruch 7, dadurch gekennzeichnet, daß das Verhältnis Carbonsäure zu Calcium etwa 2:1 Mol beträgt.

**10.** Verwendung der Zink-, Blei-, Calcium-Salze oder der zwei oder drei dieser Metalle enthaltenden Salze

gemäß den Ansprüchen 1 bis 9 als Korrosionsschutzmittel in nichtwäßrigen und wäßrigen Anstrichsystemen.

**Patentansprüche für folgenden Vertragsstaat : ES**

1. Verfahren zur Herstellung von Zink-, Blei-, Calcium-Salzen oder von zwei oder drei dieser Metalle enthaltenden gemischten Salzen von Carbonsäuren, dadurch gekennzeichnet, daß man Carbonsäuren der allgemeinen Formel

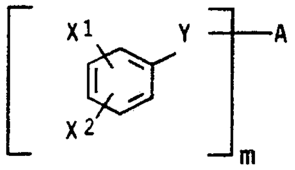

(I),

in der A für einen Rest der Formel

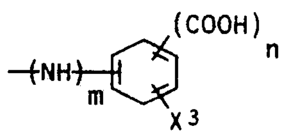

oder

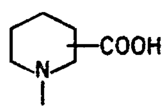

,

(II) (III)

Y für -CO- oder $-SO_2-$,
$X^1$ für $C_1$- bis $C_8$-Alkyl, $C_1$- bis $C_8$-Alkoxy oder Halogen,
$X^2$ für Wasserstoff, $C_1$- bis $C_8$-Alkyl oder Halogen,
$X^3$ für Wasserstoff, Nitro oder Halogen,
m für 1 oder 2 und
n für 1 oder 2 stehen, und wobei $X^1$ auch Wasserstoff sein kann, wenn A entweder ein Rest der Formel (III) oder $X^3$ Nitro oder m = 2 ist,

entweder mit Zink-, Blei- und/oder Calciumoxid oder mit löslichen Zink-, Blei- und/oder Calciumsalzen in Gegenwart äquivalenter Mengen an Basen im wäßrigen Medium umsetzt und die Salze von (I) isoliert.

2. Verfahren gemäß Anspruch 1, dadurch gekennzeichnet, daß in der Formel der Carbonsäure Y für $-SO_2$ steht.

3. Verfahren gemäß Anspruch 1 oder 2, dadurch gekennzeichnet, daß in der Formel der Carbonsäure $X^1$ für $C_1$- bis $C_4$-Alkyl, $C_1$- bis $C_4$-Alkoxy oder Chlor, $X^2$ für Wasserstoff und n und m jeweils für 1 stehen.

4. Verfahren gemäß Anspruch 3, dadurch gekennzeichnet, daß in der Formel der Carbonsäure $X^1$ für Methyl oder Ethyl steht.

5. Verfahren gemäß Anspruch 1, 2, 3 oder 4, dadurch gekennzeichnet, daß das Verhältnis Carbonsäure (I) zu Zink und/oder Blei etwa 1:1 Mol beträgt.

6. Verfahren gemäß Anspruch 1, 2, 3 oder 4, dadurch gekennzeichnet, daß das Verhältnis Carbonsäure (I) zu Calcium etwa 2:1 Mol beträgt.

7. Verfahren gemäß Anspruch 1, dadurch gekennzeichnet, daß man Carbonsäuren der Formel

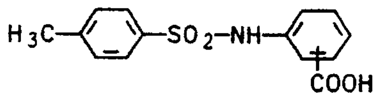 ,

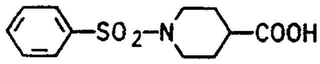 ,

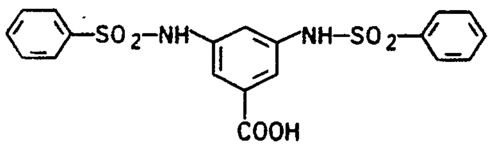

oder Gemische davon umsetzt.

8. Verfahren gemäß Anspruch 7, dadurch gekennzeichnet, daß das Verhältnis Carbonsäuren zu Zink und/oder Blei etwa 1:1 Mol beträgt.

9. Verfahren gemäß Anspruch 8, dadurch gekennzeichnet, daß das Verhältnis Carbonsäuren zu Calcium etwa 2:1 Mol beträgt.

10. Verwendung der gemäß dem Verfahren der Ansprüche 1 bis 9 erhältlichen Salze als Korrosionsschutzmittel in nichtwäßrigen und wäßrigen Anstrichsystem.

**Claims**
**Claims for the following Contracting States : AT, BE, CH, DE, FR, GB, IT, LI, NL**

1. A zinc salt, lead salt or calcium salt, or a mixed salt containing two or three of these metals, of a carboxylic acid of the formula

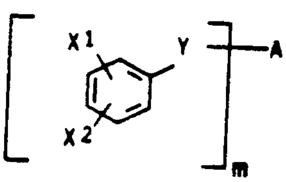 (I),

where A is a radical of the formula

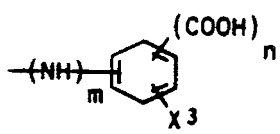 or 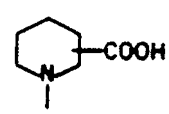 ,

(II) (III)

Y is -CO- or $-SO_2-$, $X^1$ is $C_1-_c8$-alkyl, $C_1$-$C_8$-alkoxy or halogen, $X^2$ is hydrogen, $C_1$-$C_8$-alkyl or halogen, $X^3$ is hydrogen, nitro or halogen, m and n are each 1 or 2, and $X^1$ may furthermore be hydrogen if A is a radical of the formula (III) or $X^3$ is nitro or m is 2.

2. A zinc salt, lead salt or calcium salt or a mixed salt thereof as claimed in claim 1, wherein Y is $-SO_2-$.

3. A zinc salt, lead salt or calcium salt or a mixed salt thereof as claimed in claim 1 or 2, wherein $X^1$ is $C_1$-$C_4$-alkyl, $C_1$-$C_4$-alkoxy or chlorine, $X^2$ is hydrogen and n and m are each 1.

4. A zinc salt, lead salt or calcium salt or a mixed salt thereof as claimed in claim 3, wherein $X^1$ is methyl

or ethyl.

5. A zinc salt, lead salt or zinc/lead salt as claimed in claim 1, 2, 3 or 4, wherein the molar ratio of carboxylic acid to zinc and/or lead is about 1 : 1.

6. A calcium salt as claimed in claim 1, 2, 3 or 4, wherein the molar ratio of carboxylic acid to calcium is about 2 : 1.

7. A zinc salt, lead salt or calcium salt or a mixed sält thereof as claimed in claim 1, wherein the carboxylic acid (I) is of the formula

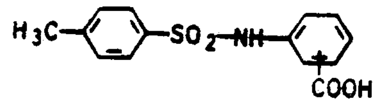
,

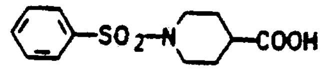
or

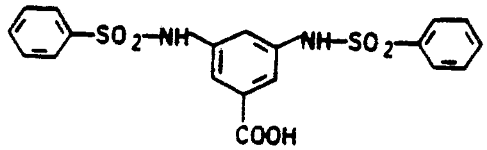

8. A zinc salt, lead salt or zinc/lead salt as claimed in claim 7, wherein the molar ratio of carboxylic acid to zinc and/or lead is about 1 : 1.

9. A calcium salt as claimed in claim 7, wherein the molar ratio of carboxylic acid to calcium is about 2 : 1.

10. Use of a zinc salt, lead salt or calcium salt or of a salt containing two or three of these metals as claimed in claims 1 to 9 as a corrosion inhibitor in nonaqueous and aqueous coating systems.

**Claims for the following Contracting State : ES**

1. A process for preparing a zinc salt, lead salt or calcium salt, or a mixed salt containing two or three of these metals, of a carboxylic acid wherein a carboxylic acid of the formula

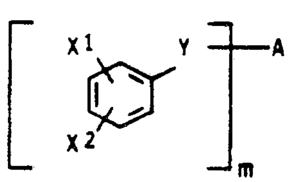
(I),

where A is a radical of the formula

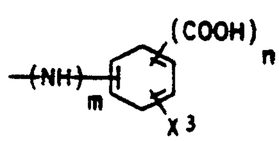
(II)

or

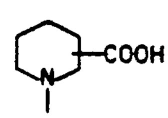
,

(III)

Y is -CO- or $-SO_2-$, $X^1$ is $C_1$-$C_8$-alkyl, $C_1$-$C_8$-alkoxy or halogen, $X^2$ is hydrogen, $C_1$-$C_8$-alkyl or halogen, $X^3$ is hydrogen, nitro or halogen, m and n are each 1 or 2, and $X^1$ may furthermore be hydrogen if A is a radical of the formula (III) or $X^3$ is nitro or m is 2, is reacted either with a zinc oxide, lead oxide or calcium oxide, or with a soluble zinc salt, lead salt or calcium salt in the presence of an equivalent amount of a base in an aqueous medium, and the salt of (I) is isolated.

**2.** A process as claimed in claim 1, wherein in the formula of the carboxlic acid Y is $-SO_2-$.

**3.** A process as claimed in claim 1 or 2, wherein in the formula of the carboxylic acid $X^1$ is $C_1$-$C_4$-alkyl, $C_1$-$C_4$-alkoxy or chlorine, $X^2$ is hydrogen is hydrogen and n and m are each 1.

**4.** A process as claimed in claim 3, wherein in the formula of the carboxylic acid $X^1$ is methyl or ethyl.

**5.** A process as claimed in claim 1, 2, 3 or 4, wherein the molar ratio of carboxylic acid (I) to zinc or lead is about 1 : 1.

**6.** A process as claimed in claim 1, 2, 3 or 4, wherein the molar ratio of carboxylic acid (I) to calcium is about 2 : 1.

**7.** A process as claimed in claim 1, wherein the carboxylic acid of the formula

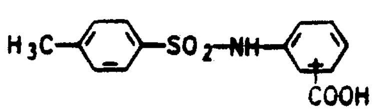

,

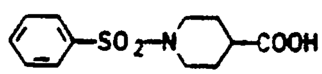

or

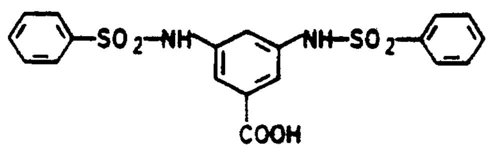

or a mixture thereof is reacted.

**8.** A process as claimed in claim 7, wherein the molar ratio of carboxylic acid to zinc or lead is about 1 : 1.

**9.** A process as claimed in claim 8, wherein the molar ratio of carboxylic acid to calcium is about 2 : 1.

**10.** Use of a salt obtainable by the process as claimed in claims 1 to 9 as a corrosion inhibitor in nonaqueous and aqueous coating sytems.

## Revendications

## Revendications pour les Etats contractants suivants : AT, BE, CH, DE, FR, GB, IT, LI, NL

**1.** Sels de zinc, de plomb, de calcium, ou sels mixtes contenant 2 ou 3 de ces métaux, d'acides carboxyliques de la formule générale

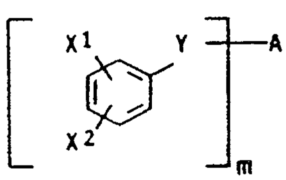

(I),

dans laquelle A représente un radical de la formule

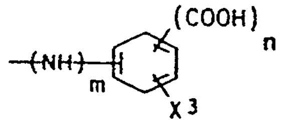 ou 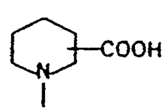 ,

(II) (III)

- Y représente le groupe -CO- ou $-SO_2-$,
- $X^1$ représente un radical alkyle en $C_1$ à $C_8$, alcoxy en $C_1$ à $C_8$, ou un atome d'halogène,
- $X^2$ représente un atome d'hydrogène, un radical alkyle en $C_1$-$C_8$ ou un atome d'halogène,
- $X^3$ représente un atome d'hydrogène, le radical nitro, ou un atome d'halogène,
- m est égal à 1 ou à 2 et
- n est égal à 1 ou à 2 et où $X^1$ peut aussi représenter un atome d'hydrogène lorsque A représente un radical de la formule (III) ou que $X^3$ représente le radical nitro ou que m est égal à 2.

2. Sels de zinc, de plomb, de cadmium, ou sels mixtes de ceux-ci, selon la revendication 1, caractérisés en ce que Y représente le radical $-SO_2$.

3. Sels de zinc, de plomb, de cadmium, ou sels mixtes de ceux-ci, selon la revendication 1 ou 2, caractérisés en ce que $X^1$ représente un radical alkyle en $C_1$ à $C_4$, alcoxy en $C_1$ à $C_4$, ou un atome de chlore, $X^2$ représente un atome d'hydrogène et n et m sont chacun égaux à 1.

4. Sels de zinc, de plomb, de cadmium, ou sels mixtes de ceux-ci, selon la revendication 3, caractérisés en ce que $X^1$ représente le radical méthyle ou éthyle.

5. Sels de zinc, de plomb, de cadmium, ou sels mixtes de ceux-ci, selon la revendication 1, 2, 3 ou 4, caractérisés en ce que le rapport de l'acide carboxylique au zinc et/ou au plomb s'élève à environ 1:1 mole.

6. Sels de calcium suivant la revendication 1, 2, 3 ou 4, caractérisés en ce que le rapport de l'acide carboxylique au calcium s'élève à environ 2:1 moles.

7. Sels de zinc, de plomb, de cadmium, ou sels mixtes de ceux-ci, selon la revendication 1, caractérisés en ce que l'acide carboxylique (I) répond aux formules

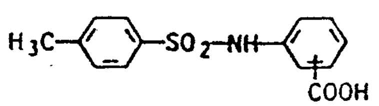 ,

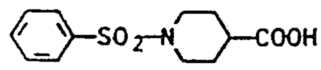 ou

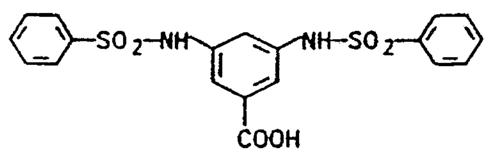

8. Sels de zinc, de plomb, ou de zinc/plomb suivant la revendication 7, caractérisés en ce que le rapport de l'acide carboxylique au zinc et/ou au plomb s'élève à environ 1:1 mole.

9. Sels de calcium suivant la revendication 7, caractérisés en ce que le rapport de l'acide carboxylique au calcium s'élève à environ 2:1 moles.

10. Utilisation des sels de zinc, de plomb, de cadmium, ou de sels contenant 2 ou 3 de ces métaux, selon les revendications 1 à 9, à titre d'agents de protection contre la corrosion dans des systèmes de peinture à base aqueuse et à base non aqueuse.

**Revendications pour l'Etat contractant suivant : ES**

1. Procédé de préparation de sels de zinc, de plomb, de calcium, ou de sels mixtes contenant 2 ou 3 de ces métaux, d'acides carboxyliques, caractérisé en ce que l'on fait réagir des acides carboxyliques de la formule générale

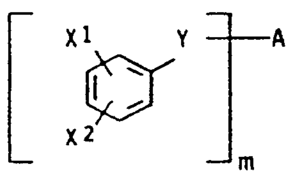

(I),

dans laquelle A représente un radical de la formule

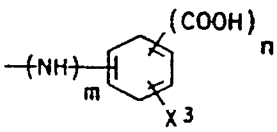

(II)

ou

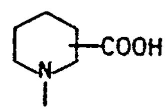

(III)

,

| | |
|---|---|
| Y | représente le groupe -CO- ou $-SO_2-$, |
| $X^1$ | représente un radical alkyle en $C_1$ à $C_8$, alcoxy en $C_1$ à $C_8$, ou un atome d'halogène, |
| $X^2$ | représente un atome d'hydrogène, un radical alkyle en $C_1$-$C_8$ ou un atome d'halogène, |
| $X^3$ | représente un atome d'hydrogène, le radical nitro, ou un atome d'halogène, |
| m | est égal à 1 ou à 2 et |
| n | est égal à 1 ou à 2 et où $X^1$ peut aussi représenter un atome d'hydrogène lorsque A représente un radical de la formule (III) ou que $X^3$ représente le radical nitro ou que m est égal à 2, |

avec de l'oxyde de zinc, de plomb et/ou de calcium, ou avec des sels de zinc, de plomb et/ou de calcium solubles, en présence de proportions équivalentes de bases, en milieu aqueux et on isole les sels des composés (I).

2. Procédé suivant la revendication 1, caractérisé en ce que Y représente $-SO_2-$ dans la formule de l'acide carboxylique.

3. Procédé suivant la revendication 1 ou 2, caractérisé en ce que $X^1$ représente un radical alkyle en $C_1$ à $C_4$, alcoxy en $C_1$ à $C_4$, ou un atome de chlore, $X^2$ représente un atome d'hydrogène et n et m sont chacun égaux à 1 dans la formule de l'acide carboxylique.

4. Procédé suivant la revendication 3, caractérisé en ce que $X^1$ représente le radical méthyle ou éthyle dans la formule de l'acide carboxylique.

5. Procédé suivant la revendication 1, 2, 3 ou 4, caractérisé en ce que le rapport de l'acide carboxylique (1) au zinc et/ou plombe s'élève à environ 1:1 mole.

6. Procédé suivant la revendication 1, 2, 3 ou 4, caractérisé en ce que le rapport de l'acide carboxylique (I) au calcium s'élève à environ 2:1 moles.

7. Procédé suivant la revendication 1, caractérisé en ce que l'on fait réagir des acides carboxyliques des formules

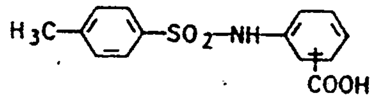

,

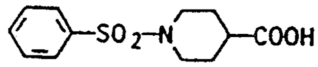

,

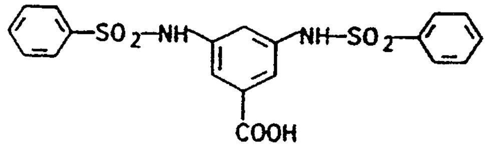

ou des mélanges de ceux-ci.

8. procédé suivant la revendication 7, caractérisé en ce que le rapport des acides carboxyliques au zinc et/ou au plomb s'élève à environ 1:1 mole.

9. Procédé suivant la revendication 8, caractérisé en ce que le rapport des acides carboxyliques au calcium s'élève à environ 2:1 moles.

10. Utilisation des sels que l'on peut obtenir selon le procédé des revendications 1 à 9 à titre d'agents de protection contre la corrosion dans des systèmes de peinture à base aqueuse et à base non aqueuse.